# EUROPEAN PATENT SPECIFICATION

(11) **EP 2 456 785 B1**
(45) Date of publication and mention of the grant of the patent: **19.11.2014**
(21) Application number: 10747517.0
(22) Date of filing: 21.07.2010
(51) Int. Cl.: C07K 14/025, C07K 14/415, C12N 15/62, A61K 38/16

(54) **VACCINES BASED ON GENETIC CHIMERA OF VIRAL AND/OR TUMORAL ANTIGENS AND VEGETABLE PROTEINS**
IMPFSTOFFE AUF DER BASIS VON GENETISCHEN CHIMÄREN VON VIRUS- UND/ODER TUMOR-ANTIGENEN UND PFLANZENPROTEINEN
VACCINS À BASE DE CHIMÈRE GÉNÉTIQUE D'ANTIGÈNES VIRAUX ET/OU TUMORAUX ET DE PROTÉINES VÉGÉTALES

(30) Priority: 21.07.2009 IT RM20090383
(43) Date of publication of application: 30.05.2012
(73) Proprietor: ENEA - ENTE PER LE NUOVE TECNOLOGIE, L'ENERGIA E L'AMBIENTE, 00196 Roma (IT); Università degli Studi dell'Aquila, 67100 L'Aquila (AQ) (IT); Istituti Fisioterapici Ospitalieri, 00128 Roma (IT); Venuti, Aido, 00048 Nettuno (RM) (IT)
(72) Inventor: VENUTI, AIdo, 00048 Nettuno (RM) (IT); FRANCONI, Rosella, 00123 Roma (IT); MASSA, Silvia, 73013 Galatina (LE) (IT); SPANO', Laura, 00161 Roma (IT)
(74) Representative: Gitto, Serena
(86) International application number: PCT/IT2010/000324
(87) International publication number: WO 2011/010337

(56) References cited:
- US-B1- 7 175 848
- MASSA SILVIA ET AL: "Antitumor activity of DNA vaccines based on the human papillomavirus-16 E7 protein genetically fused to a plant virus coat protein" HUMAN GENE THERAPY, MARY ANN LIEBERT, NEW YORK ,NY, US, vol. 19, no. 4, 1 April 2008 (2008-04-01), pages 354-364, XP009129816 ISSN: 1043-0342
- ZAROVNI N ET AL: "Saporin as a novel suicide gene in anticancer gene therapy" CANCER GENE THERAPY, NORWALK, CT, US, vol. 14, no. 2, 1 February 2007 (2007-02-01), pages 165-173, XP002565399 ISSN: 0929-1903 [retrieved on 2006-09-29]
- FRAZER IAN H: "Prevention of cervical cancer through papillomavirus vaccination." NATURE REVIEWS IMMUNOLOGY, vol. 4, no. 1, January 2004 (2004-01), pages 46-54, XP002575094 ISSN: 1474-1733
- MATHEW MRUDULA ET AL: "Humanized immunotoxins: a new generation of immunotoxins for targeted cancer therapy." CANCER SCIENCE AUG 2009, vol. 100, no. 8, 19 May 2009 (2009-05-19), pages 1359-1365, XP002575095 ISSN: 1349-7006

### SEQUENCE LISTING

<110> ENEA Università degli Studi dell'Aquila Istituti Fisioterapici ospitalieri
<120> vaccines based on genetic chimera of viral and/or tumoral antigens and vegetable proteins
<130> PCT27948
<150> RM2009A000383
   <151> 2009-08-21
<160> 42
<170> PatentIn version 3.5
<210> 1
   <211> 42
   <212> DNA
   <213> artificial
<220>
   <223> forward primer for the mutagenesis of saporine
<400> 1
   ctattcagat gacggctaag gcagcgcagt ttaggtacat ac 42
<210> 2
   <211> 43
   <212> DNA
   <213> artificial
<220>
   <223> reverse primer for the mutagenesis of saporine
<400> 2
   gtatgtacct aaactgcgct gccttagccg tcatctgaat agc 43
<210> 3
   <211> 18
   <212> DNA
   <213> artificial
<220>
   <223> wild type E7 gene
<400> 3
   gatctctact gttatgag 18
<210> 4
   <211> 6
   <212> PRT
   <213> artificial
<220>
   <223> motif codified by SEQ ID NO:3
<400> 4
   Asp Leu Tyr Cys Tyr Glu
   1 5
<210> 5
   <211> 18
   <212> DNA
   <213> artificial
<220>
   <223> mutated E7 gene
<400> 5
   ggtctctacg gttatggg 18
<210> 6
   <211> 6
   <212> PRT
   <213> artificial
<220>
   <223> motif codified by SEQ ID NO:5
<400> 6
   Gly Leu Tyr Gly Tyr Gly
   1 5
<210> 7
   <211> 36
   <212> DNA
   <213> artificial
<220>
   <223> wild type saporine gene
<400> 7
   attcagatga cggctgaggc agcgcgattt aggtac 36
<210> 8
   <211> 12
   <212> PRT
   <213> artificial
<220>
   <223> motif codified by SEQ ID NO:7
<400> 8
   Ile Gln Met Thr Ala Glu Ala Ala Arg Phe Arg Tyr
   1 5 10
<210> 9
   <211> 36
   <212> DNA
   <213> artificial
<220>
   <223> mutated saporine gene
<400> 9
   attcagatga cggctaaggc agcgcagttt aggtac 36
<210> 10
   <211> 12
   <212> PRT
   <213> artificial
<220>
   <223> motif codified by SEQ ID NO:9
<400> 10
   Ile Gln Met Thr Ala Lys Ala Ala Gln Phe Arg Tyr
   1               5                   10
<210> 11
   <211> 12
   <212> PRT
   <213> artificial
<220>
   <223> catalytic site partial aminoacid sequence of ricin A chain
<400> 11
   Ile Gln Met Ile Ser Glu Ala Ala Arg Phe Gln Tyr
   1               5                   10
<210> 12
   <211> 12
   <212> PRT
   <213> artificial
<220>
   <223> catalytic site partial aminoacid sequence of apoplastic saporine
<400> 12
   Ile Gln Met Thr Ala Glu Ala Ala Arg Phe Arg Tyr
   1               5                   10
<210> 13
   <211> 12
   <212> PRT
   <213> artificial
<220>
   <223> catalytic site partial amonoacid sequence of PAP
<400> 13
   Ile Gln Met Ile Ser Glu Ala Ala Arg Phe Lys Tyr
   1               5                   10
<210> 14
   <211> 879
   <212> DNA
   <213> artificial
<220>
   <223> saporine apoplastic sequence
<400> 14
   atgaagatat atgttgtagc cacaatagca tggatcctgc ttcaattttc agcttggaca 60
   acaactgatg cggtcacatc aatcacatta gatctagtaa atccgaccgc gggtcaatac 120
   tcatcttttg tggataaaat ccgaaacaac gtaaaggatc caaacctgaa atacggtggt 180
   accgacatag ccgtgatagg cccaccttct aaagaaaaat tccttagaat taatttccaa 240
   agttcccgag gaacggtctc acttggccta aaacgcgata acttgtatgt ggtcgcgtat 300
   cttgcaatgg ataacacgaa tgttaatcgg gcatattact tcagatcaga aattacttcc 360
   gccgagttaa ccgccctttt cccagaggcc acaactgcaa atcagaaagc tttagaatac 420
   acagaagatt atcagtcgat tgaaaagaat gcccagataa cacaaggaga tcaaagtaga 480
   aaagaactcg ggttggggat tgacttactt tcaacgtcca tggaagcagt gaacaagaag 540
   gcacgtgtgg ttaaagacga agctagattc cttcttatcg ctattcagat gacggctgag 600
   gcagcgcgat ttaggtacat acaaaacttg gtaatcaaga actttcccaa caagttcaac 660
   tcggaaaaca aagtgattca gtttgaggtt aactggaaaa aaatttctac ggcaatatac 720
   ggggatgcca aaaacggcgt gtttaataaa gattatgatt tcgggtttgg aaaagtgagg 780
   caggtgaagg acttgcaaat gggactcctt atgtatttgg gcaaaccaaa gtcgtcaaac 840
   gaggcgaatt ctactgctta cgccactacg gtcctctga 879
<210> 15
   <211> 297
   <212> DNA
   <213> artificial
<220>
   <223> HPV16-E7 sequence
<400> 15
   atgcatggag atacacctac attgcatgaa tatatgttag atttgcaacc agagacaact 60
   gatctctact gttatgagca attaaatgac agctcagagg aggaggatga aatagatggt 120
   ccagctggac aagcagaacc ggacagagcc cattacaata ttgtaacctt ttgttgcaag 180
   tgtgactcta cgcttcggtt gtgcgtacaa agcacacacg tagacattcg tactttggaa 240
   gacctgttaa tgggcacact aggaattgtg tgccccatct gttctcagaa accataa 297
<210> 16
   <211> 48
   <212> DNA
   <213> artificial
<220>
   <223> forward primer for the mutagenesis of E7 gene
<400> 16
   caacaagaga caactggtct ctacggttat gggcaattaa atgacagc 48
<210> 17
   <211> 49
   <212> DNA
   <213> artificial
<220>
   <223> reverse primer for the mutagenesis of E7 gene
<400> 17
   gctgtcattt aattgcccat aaccgtagag accagttgtc tctggttgc 49
<210> 18
   <211> 42
   <212> DNA
   <213> artificial
<220>
   <223> forward primer for the mutagenesis of saporine gene
<400> 18
   ctattcagat gacggctaag gcagcgcagt ttaggtacat ac 42
<210> 19
   <211> 43
   <212> DNA
   <213> artificial
<220>
   <223> reverse primer for the mutagenesis of saporine gene
<400> 19
   gtatgtacct aaactgcgct gccttagccg tcatctgaat agc 43
<210> 20
   <211> 48
   <212> DNA
   <213> artificial
<220>
   <223> amplification forward primer of mutated E7 gene
<400> 20
   ggccatcgat ctgcaggcat gcatcatgca tggagataca cctacatt 48
<210> 21
   <211> 39
   <212> DNA
   <213> artificial
<220>
   <223> reverse amplification primer of mutated E7 gene
<400> 21
   ggccctcgag gtcgacttat ggtttctgag aacagatgg 39
<210> 22
   <211> 50
   <212> DNA
   <213> artificial
<220>
   <223> amplification forward primer of mutated saporine gene
<400> 22
   ggccatcgat ctgcaggcat gcatcatggt cacatcaatc acattagatc 50
<210> 23
   <211> 38
   <212> DNA
   <213> artificial
<220>
   <223> amplification reverse primer of mutated saporine gene
<400> 23
   ggccctcgag gtcgactcac tttggtttgc ccaaatac 38
<210> 24
   <211> 39
   <212> DNA
   <213> artificial
<220>
   <223> amplification reverse primer of mutated E7 gene
<400> 24
   taatgtgatt gatgtgactg gtttctgaga acagatggg 39
<210> 25
   <211> 40
   <212> DNA
   <213> artificial
<220>
   <223> amplification forward primer of mutated saporine gene
<400> 25
   atctgttctc agaaaccagt cacatcaatc acattagatc 40
<210> 26
   <211> 42
   <212> DNA
   <213> artificial
<220>
   <223> amplification reverse primer of mutated saporine gene
<400> 26
   tgtaggtgta tctccatgct ttggtttgcc caaatacata ag 42
<210> 27
   <211> 40
   <212> DNA
   <213> artificial
<220>
   <223> amplification forward primer of mutated E7 gene
<400> 27
   tatttgggca aaccaaagca tggagataca cctacattgc 40
<210> 28
   <211> 48
   <212> DNA
   <213> artificial
<220>
   <223> amplification forward primer of mutated saporine gene
<400> 28
   ggccatcgat ctgcaggcat gcatcatgaa gatatatgtt gtagccac 48
<210> 29
   <211> 292
   <212> PRT
   <213> artificial
<220>
   <223> saporine apoplastic aminoacidic sequence
<400> 29
   Met Lys Ile Tyr Val Val Ala Thr Ile Ala Trp Ile Leu Leu Gln Phe
   1               5                   10                  15
   Ser Ala Trp Thr Thr Thr Asp Ala Val Thr Ser Ile Thr Leu Asp Leu
               20                  25                  30
   Val Asn Pro Thr Ala Gly Gln Tyr Ser Ser Phe Val Asp Lys Ile Arg
           35                  40                  45
   Asn Asn Val Lys Asp Pro Asn Leu Lys Tyr Gly Gly Thr Asp Ile Ala
       50                  55                  60
   Val Ile Gly Pro Pro Ser Lys Glu Lys Phe Leu Arg Ile Asn Phe Gln
   65                  70                  75                  80
   Ser Ser Arg Gly Thr Val Ser Leu Gly Leu Lys Arg Asp Asn Leu Tyr
                   85                  90                  95
   Val Val Ala Tyr Leu Ala Met Asp Asn Thr Asn Val Asn Arg Ala Tyr
               100                 105                 110
   Tyr Phe Arg Ser Glu Ile Thr Ser Ala Glu Leu Thr Ala Leu Phe Pro
           115                 120                 125
   Glu Ala Thr Thr Ala Asn Gln Lys Ala Leu Glu Tyr Thr Glu Asp Tyr
       130                 135                 140
   Gln Ser Ile Glu Lys Asn Ala Gln Ile Thr Gln Gly Asp Gln Ser Arg
   145                 150                 155                 160
   Lys Glu Leu Gly Leu Gly Ile Asp Leu Leu Ser Thr Ser Met Glu Ala
                   165                 170                 175
   Val Asn Lys Lys Ala Arg Val Val Lys Asp Glu Ala Arg Phe Leu Leu
               180                 185                 190
   Ile Ala Ile Gln Met Thr Ala Glu Ala Ala Arg Phe Arg Tyr Ile Gln
           195                 200                 205
   Asn Leu Val Ile Lys Asn Phe Pro Asn Lys Phe Asn Ser Glu Asn Lys
       210                 215                 220
   Val Ile Gln Phe Glu Val Asn Trp Lys Lys Ile Ser Thr Ala Ile Tyr
   225                 230                 235                 240
   Gly Asp Ala Lys Asn Gly Val Phe Asn Lys Asp Tyr Asp Phe Gly Phe
                   245                 250                 255
   Gly Lys Val Arg Gln Val Lys Asp Leu Gln Met Gly Leu Leu Met Tyr
   260 265 270
   Leu Gly Lys Pro Lys Ser Ser Asn Glu Ala Asn Ser Thr Ala Tyr Ala
   275 280 285
   Thr Thr Val Leu
   290
<210> 30
   <211> 98
   <212> PRT
   <213> artificial
<220>
   <223> HPV16-E7 aminoacidic sequence
<400> 30
   Met His Gly Asp Thr Pro Thr Leu His Glu Tyr Met Leu Asp Leu Gln
   1 5 10 15
   Pro Glu Thr Thr Asp Leu Tyr Cys Tyr Glu Gln Leu Asn Asp Ser Ser
   20 25 30
   Glu Glu Glu Asp Glu Ile Asp Gly Pro Ala Gly Gln Ala Glu Pro Asp
   35 40 45
   Arg Ala His Tyr Asn Ile Val Thr Phe Cys Cys Lys Cys Asp Ser Thr
   50 55 60
   Leu Arg Leu Cys Val Gln Ser Thr His Val Asp Ile Arg Thr Leu Glu
   65 70 75 80
   Asp Leu Leu Met Gly Thr Leu Gly Ile Val Cys Pro Ile Cys Ser Gln
   85 90 95
   Lys Pro
<210> 31
   <211> 16
   <212> PRT
   <213> artificial
<220>
   <223> catalytic site partial aminoacid sequence of luffin a
<400> 31
   Thr Val Ala Val Asp Val Ser Gln Leu Tyr Ile Met Gly Tyr Leu Val
   1 5 10 15
<210> 32
   <211> 16
   <212> PRT
   <213> artificial
<220>
   <223> catalytic site partial aminoacid sequence of luffin b
<400> 32
   Thr Met Ala Ile Asp Val Thr Asn Val Tyr Ile Met Gly Tyr Leu Val
   1               5                   10                  15
<210> 33
   <211> 16
   <212> PRT
   <213> artificial
<220>
   <223> catalytic site partial aminoacid sequence of ricin chain A
<400> 33
   Thr Leu Ala Leu Asp Val Thr Asn Ala Tyr Val Val Gly Tyr Arg Ala
   1               5                   10                  15
<210> 34
   <211> 16
   <212> PRT
   <213> artificial
<220>
   <223> catalytic site partial aminoacid sequence of saporine S6
<400> 34
   Ser Leu Gly Leu Lys Arg Asp Asn Leu Tyr Val Val Ala Tyr Leu Ala
   1               5                   10                  15
<210> 35
   <211> 16
   <212> PRT
   <213> artificial
<220>
   <223> catalytic site partial aminoacid sequence of tricosanthin
<400> 35
   Ser Val Ala Ile Asp Val Thr Asn Val Tyr Ile Met Gly Tyr Arg Ala
   1               5                   10                  15
<210> 36
   <211> 20
   <212> PRT
   <213> artificial
<220>
   <223> catalytic site partial aminoacid sequence of luffin a
<400> 36
   Ala Ala Ala Phe Leu Val Ile Leu Gln Thr Thr Ala Glu Ala Ser Arg
   1               5                   10                  15
   Phe Lys Tyr Ile
               20
<210> 37
   <211> 20
   <212> PRT
   <213> artificial
<220>
   <223> catalytic site partial aminoacid sequence of luffin b
<400> 37
   Ala Ala Ala Phe Leu Val Ile Leu Gln Thr Thr Ala Glu Ala Ser Arg
   1               5                   10                  15
   Phe Lys Tyr Ile
               20
<210> 38
   <211> 20
   <212> PRT
   <213> artificial
<220>
   <223> catalytic site partial aminoacid sequence of ricin chain A
<400> 38
   Ala Arg Ser Phe Ile Ile Cys Ile Gln Met Ile Ser Glu Ala Ala Arg
   1               5                   10                  15
   Phe Gln Tyr Ile
               20
<210> 39
   <211> 20
   <212> PRT
   <213> artificial
<220>
   <223> catalytic site partial aminoacid sequence of saporine S6
<400> 39
   Ala Arg Phe Leu Leu Ile Ala Ile Gln Met Thr Ala Glu Ala Ala Arg
   1 5 10 15
   Phe Arg Tyr Ile
   20
<210> 40
   <211> 20
   <212> PRT
   <213> artificial
<220>
   <223> catalytic site partial aminoacid sequence of tricosanthin
<400> 40
   Ala Ser Ala Leu Met Val Leu Ile Gln Ser Thr Ser Glu Ala Ala Arg
   1 5 10 15
   Tyr Lys Phe Ile
   20
<210> 41
   <211> 16
   <212> PRT
   <213> artificial
<220>
   <223> catalytic site partial aminoacid sequence of PAP
<400> 41
   Thr Leu Met Leu Arg Arg Asn Asn Leu Tyr Val Met Gly Tyr Ser Asp
   1 5 10 15
<210> 42
   <211> 20
   <212> PRT
   <213> artificial
<220>
   <223> catalytic site partial aminoacid sequence of PAP
<400> 42
   Ala Glu Phe Leu Leu Val Ala Ile Gln Met Ile Ser Glu Ala Ala Arg
   1 5 10 15
   Phe Lys Tyr Ile
   20

## Description

The present invention refers to vaccines based on viral and/or tumoral antigens and vegetal proteins. More particularly, the present invention refers to genetic or protein therapeutic and prophylactic vaccines based on chimeras obtained by gene fusion of ribosome inhibiting proteins (RIP) without enzymatic activity as a result of treatment with viral/tumoral antigens, as for example Human Papilloma Virus E7 (HPV).

Gene therapy, as for example gene vaccine development based on administration of DNA in form of attenuated viruses or plasmids, is considered a complementary or alternative strategy to conventional anti-cancer therapies aiming to overcome the limits of the latter. One of the promises of such an approach consists of tumour elimination by administration of immunomodulating molecules suitable to promote the immune system activation. Immunomodulation can be also obtained by means of the administration of protein molecules (protein vaccines).

High risk Human Papilloma Viruses (HPV) (HR-HPV, mainly HPV-16 and HPV-18) are the etiologic agents of uterus cervix invasive cancer, i.e. first and third cancer female death in the developing countries (500000 new cases every year, half thereof fatal) and in worldwide female population, respectively. HPV anogenital infections display high rate "clearance" as a result of an effective immune response against the infection. However where the infection becomes latent, lesions are developed potentially progressing up to tumoral transformation of which E6 and E7 viral oncoproteins are mainly responsible, which on turn represent "tumour-specific" antigens.

Now two prevention anti-HPV vaccines (GARDASIL ™, Merck; CERVARIX ™, GSK) are available. Due to high cost and latency long period from infection to tumour appearance, the advantages of prophylactic vaccination in terms of incidence reduction of pre-invasive and invasive pathology will be evaluable only after tens of years, provided the protective vaccination has been carried out now on a worldwide basis.

In order to overcome such a temporal gap, a therapeutic vaccine development is necessary for the treatment of already present oncologic lesions in affected subjects and already affected or age reason prophylaxis program excluded women. Such vaccines aim to be conventional treatment co-adjuvants and elicit a specific cell-mediated immune response suitable to eliminate quickly the already developing infection and also protect the organism from possible future virus exposures.

Ideal candidates for therapeutic vaccine development are HPV E6 and E7 oncoproteins since the same are *"tumour-specific antigens".* Some recombinant E7 protein based therapeutic vaccines are currently under clinical 'trial' step. Recently it is in development phase a HPV-16 E6 and E7 expression based vaccine (and an adjuvant) by an attenuated vaccine virus.

From some years the authors of the present invention are involved in research projects aiming the effective therapeutic vaccine development against the HPV induced tumours. Particularly, studies have demonstrated that plants represent an optimal system for then production of HPV-16 E7 protein based therapeutic vaccines (Franconi *et al.,* 2002; Franconi *et al.,* 2006; Franconi R., *et al.,* 2001; 'Vaccini a subunità e procedimenti per la loro produzione' RM2001A001332, Brevetto Europeo n. 1401493; Franconi & Venuti, 2006; Massa *et al.*, 2007; Venuti *et al.,* 2009). In fact, HPV-16 E6 and E7 oncoproteins are responsible of the appearance and maintenance of the transformed state, representing therefore an appropriate target for the therapeutic vaccine development. Various HPV-16 E7 oncoprotein based vaccine formulations have been tested in animal models and some have subjected to phase II and III clinical 'trials' (Frazer, 2004; Ma *et al.* 2010). Many of these potential HPV therapeutic vaccines have been demonstrated suitable to eradicate the tumour in animal models and elicit a specific cell-mediated immune response in humans.

The development of innovative and increasingly safer vaccines is frequently based on well characterized antigens, particularly highly purified proteins or synthetic peptides; an approach of this type, however, is frequently compromised due to the fact that antigens, when administrated alone, are insufficiently immunogenic. In general terms, in fact, the attempts to promote and develop an HPV successful therapeutic vaccination have been limited due insufficient presentation of viral antigens - which are expressed at low levels - and also due to insufficient traffic of effector T-cells at not inflamed sites. For this reason the adjuvant use has been demonstrated up to now indispensable in order to guarantee therapy effectiveness.

In the light of above it is therefore apparent the need to provide for new HPV vaccines suitable to overcome the disadvantages of known prior art vaccines.

A complementary approach to the adjuvant use involves the antigen immunogenicity enhancement through fusion thereof with proteins having established immunogenic activity. It has been already demonstrated by the authors of the present invention that the fusion of potato Virus X enclosing protein (PVX-CP) with HPV-16 E7 oncoprotein (and mutant lacking of oncogenic activity, E7GGG), when supplied within a genetic vaccination, results in a marked increment of the immune antigen-specific response suitable to protect rats from tumour development (Massa *et al.,* 2008). It has been also demonstrated again by the authors of the present invention that E7GGG fusion with a bacterial origin stable protein ("lichenase") produced and purified from plant is suitable to protect animals from tumour development (Mass et *al.,* 2007).

The ribosome inhibiting proteins (RIPs) represent a vegetal origin enzyme large family suitable to inactivate ribosomes catalytically, as result of elimination of purines in 23/25/28 S ribosome RNAs, resulting in protein synthesis block and cell death. Although it is known for a long time the toxicity of the vegetal species producing these proteins and use thereof by humans, only during the last years the scientific interest about this group of enzymes has developed, particularly for potential use in medicine, in order provide targeted toxins (Stirpe, 2004; Hartley & Lord 2004; Ng, *et al.* 2010). RIP cytotoxic effectiveness, in fact, can be exploited in order to obtain immunotoxins, hybrid molecules wherein RIPs are conjugated to specific ligands (generally monoclonal antibodies) suitable for the delivery thereof to determined cellular targets. However, it has been proved that extended therapies based on repeated administrations of these extraneous proteins induce a strong humoral type immunity response resulting in a reduction of average life-time and cytoxicity inhibition (Frankel, 2004, Szalai *et al.,* 2005). The modification of immunotoxin structure, including the generation of punctiform mutants, seems to represent an appropriate instrument in order the immunogenicity thereof to be modulated (Cheung, et *al.* 2004).

Saporin is type 1 RIP, i.e. polypeptide single chain, molecular weight about 30 kDa, occurring also in *Saponaria officinalis* leaves, in addition to roots and seeds thereof. Seed SO6 isoform has been used widely for immunotoxin preparation by taking advantage of cytotoxic activity directed against specific target cells, using tumour-specific ligands (for example antibodies). SAP displays a good resistance to derivatization and conjugation processes (Santanchè et *al.,* 1997), poor non-specific toxicity and high catalytic activity within cytoplasm (Ferreras, *et al.* 1993).

The authors of the present invention have now prepared new therapeutic and prophylactic vaccines against high risk (HR-HPV, mainly HPV-16) Human Papilloma Viruses (HPV) with increased immunogenicity and effectiveness, also without adjuvant, based on the fusion of tumour HPV-16 E7 antigen and catalytic site mutated RIP Saporin (SAP-KQ).

Vaccines according to the present invention take advantage of RIP properties which in the case of therapies based on immunotoxin use are considered disadvantageous (Stirpe & Battelli, 2006), while, in the case of the therapeutic and prophylactic vaccines represent an enhancing effectiveness tool for said vaccines.

Rip interesting properties are as below:
1. immunostimulating activity: suitable to increase an immune response specific against the antigen the molecule is bound to. Mutations at SAP catalytic site (for example SAP-KQ> E176K, R179Q) or deletion mutants (Pittaluga, et *al.* 2005; Zarovni *et al.* 2007) result in a molecule not longer toxic, unable in ribosome inhibiting, meanwhile immunostimulating properties are conserved;
2. ability to induce inflammatory reactions: cytokine release is very important in order the immune system to be activated (Zhao et al., 1999);
3. vegetal origin: induction of autoimmune responses is prevented;
4. ability to induce apoptosis (Bergamaschi et *al.,* 1996);;
5. thermodynamic stability: suitable to confer greater stability to other proteins the molecule has been fused with.

Thanks to the determination of amino acid sequence of up to now purified and/or cloned RIPs, three-dimensional structure elucidation of some thereof, mutagenesis site-specific experiments, it has been possible the catalytic mechanism thereof to be explained.

The comparison of type 1 and 2 RIP primary structure has evidenced that there are many invariant and highly conserved amino acid residues (see below). Particularly, there are four invariant residues occurring in catalytic site of all RIPs: Tyr 80, Tyr 123, Glu 177, Arg 180 (positions are referred to ricin A chain). A common role for these residues is such as for tyrosine units, which through aromatic rings allow substrate to be presented to catalytic site (the adenine) in an energetically favourable conformation.

Amino acid sequences of some RIPs in catalytic site involved regions are listed below:
Luffin a (61) TVAVDVSQLYIMGYLV (77) (SEQ ID NO: 31)
   Luffin b (61) TMAIDVTNVYIMGYLV (77) (SEQ ID NO: 32)
   Ricin A (71) TLALDVTNAYVVGYRA (87) (SEQ ID NO: 33)
   chain
   Saporin S6 (63) SLGLKRDNLYVVAYLA (79) (SEQ ID NO: 34)
   Tricosanthin (61) SVAIDVTNVYIMGYRA (77) (SEQ ID NO: 35)
   PAP (63) TLMLRRNNLYVMGYSD (79) (SEQ ID NO: 41)
Luffin a (147) AAAFLVILQTTAEASRFKYI (166) (SEQ ID NO: 36)
   Luffin b (147) AAAFLVILQTTAEASRFKYI (166) (SEQ ID NO: 37)
   Ricin A (165) ARSFIICIQMISEAARFQYI (184) (SEQ ID NO: 38)
   chain
   Saporin S6 (164) ARFLLIAIQMTAEAARFRYI (183) (SEQ ID NO: 39)
   Tricosanthin (148) ASALMVLIQSTSEAARYKFI (167) (SEQ ID NO: 40)
   PAP (164) AEFLLVAIQMISEAARFKYI (183) (SEQ ID NO: 42)

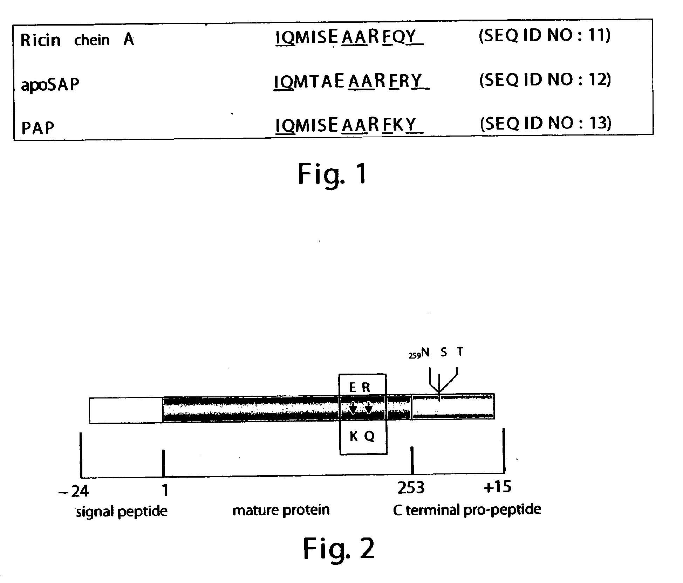

Particularly genetic chimeras for the production of retinoblastoma binding region mutated (E7GGG, not longer oncogenic, as described in Mass et *al.,* 2008) SAP fused, leaf deriving apoplastic isoform (Acc. No. DQ105520), cloned from *Saponaria officinalis* plant, as described in Tartarini, et *al.* 2010, also the latter presenting two mutated positions in catalytic site (SAP-KQ), 16 human papilloma virus (HPV-16, Acc. No. KO2718) E7 protein, have been carried out. Particularly mutagenesis involved two well conserved catalytic site amino acid residues, both in 1 and 2 type RIPs: glu 176 and arg 179 (Fig. 1, in block letter) (Frankel *et al. ,* 1990; Hur *et al.,* 1995) which have been mutated to lysine and glutamine, (E176K, R179Q), respectively using QuickChange Situated Mutagenesis' (Stratagene) kit, in gene encoding for saporin apoplastic localization leaf isoform, obtaining apoSAP-KQ mutant (Fig. 2).

Mutagenesis used primers have been as below:
*For mut KQ (42 mer):* 5' CT ATT CAG ATG ACG GCT AAG GCA GCG CAG TTT AGG TAC ATA C *3*' (**SEQ ID NO: 1**)
*Rev mut KQ (43 mer):* 5' G TAT GTA CCT AAA CTG CGC TGC CTT AGC CGT CAT CTG AAT AGC 3' (**SEQ ID NO: 2**)

It is already literature known that for both seed saporin isoform (Sol1) and genome clone (SAP3), E176K, R179Q double mutant proves to be not toxic both *in vitro -* protein synthesis inhibition (Sol1 - Marshall et *al.,* 2010) and *in vivo -* cytotoxicity on culture cell lines (SAP3 - Zarovni et *al.,* 2007).

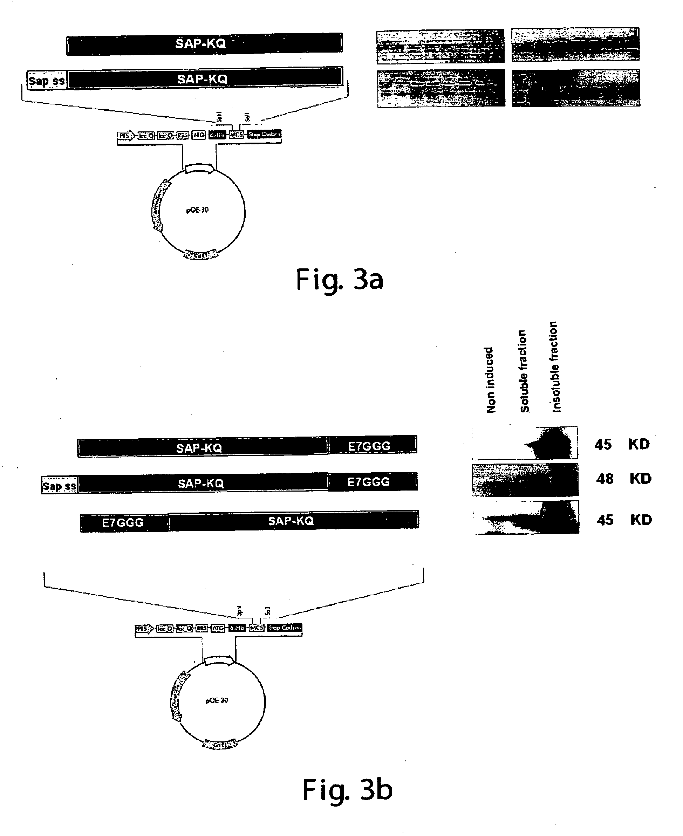

Various gene fusions (Fig. 3) have carried out. In particular E7GGG gene has been fused 'in frame' at 3' and/or 5' end of apoSAP-KQ gene, whether containing a signal peptide (ss) at N-terminal in order to allow the protein to be directed to secretion pathway and without C-terminal peptide, which is normally removed in protein mature form. SAP apoplastic signal peptide (apoSAPss) has been used. Further directing signals deriving from *apoSAP* sequence (for example C-terminal region) or other available saporin isoform genes, or other vegetal known proteins, can be used in order the yield and/or recovery (purification) of the fusion product to be optimized. Moreover, other SAP gene mutants can be obtained in order the catalytic activity to be eliminated (Bonini *et al.,* 2006) and/or minimum molecular weight form, suitable to maintain immunogenic properties, to be characterised.

apoSAP-KQ and ssapoSAP-KQ single genes and fusions have been cloned in a vector for the expression in *Escherichia coli* (pQE30, Fig. 3a, 3b). This plasmid comprises a '6xHis-tag' sequence at 5' cloning site and allows the expression of proteins provided with an histidine tail at C-terminus allowing the purification thereof using Ni-NTA affinity chromatography technology. Thus in addition to prove the possibility proteins of interest to be expressed (also in mammalian cells) and reference standards to be obtained, the possibility the vaccine of interest also in protein form, taking advantage of production prokaryotic systems, to be produced is evaluated.

SAP-KQ and ssSAP-KQ proteins have been expressed and purified (Fig. 3a). E7GGG-SAP-KQ, SAP-KQ-E7GGG and ssSAP-KQ-E7GGG fusions have been expressed obtaining products with expected molecular weight (Fig. 3b).

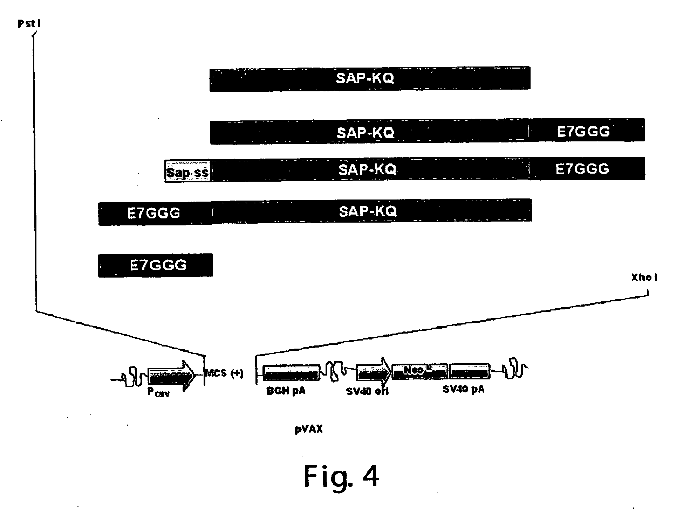

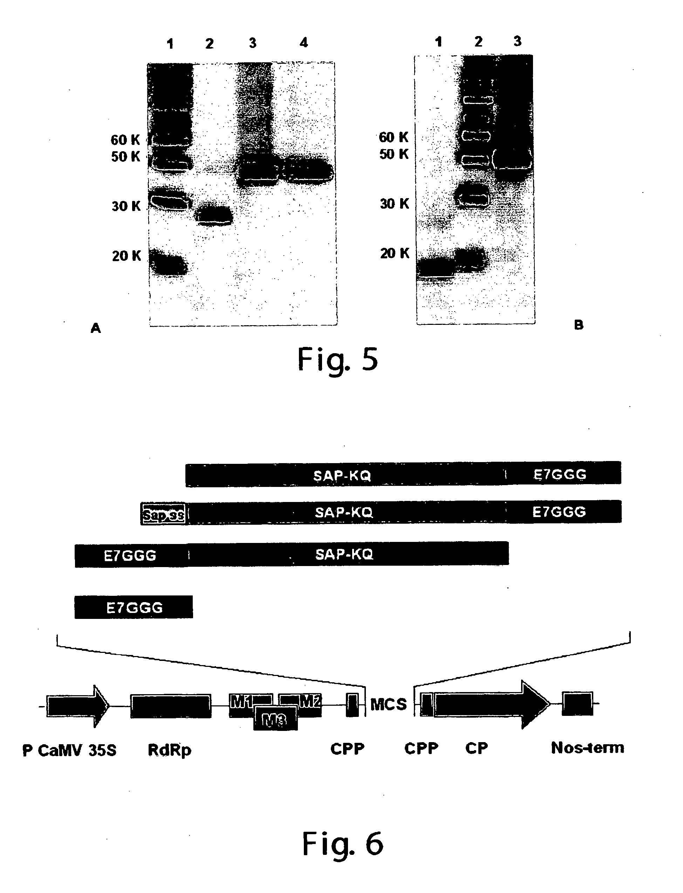

Fusion products have been cloned in an expression vector in mammalian cells (pVAX, Fig. 4). Mammalian cells (i.e. HEK 293) allow the production of expected proteins. Transfection results with some of obtained constructs are shown in Fig. 5.

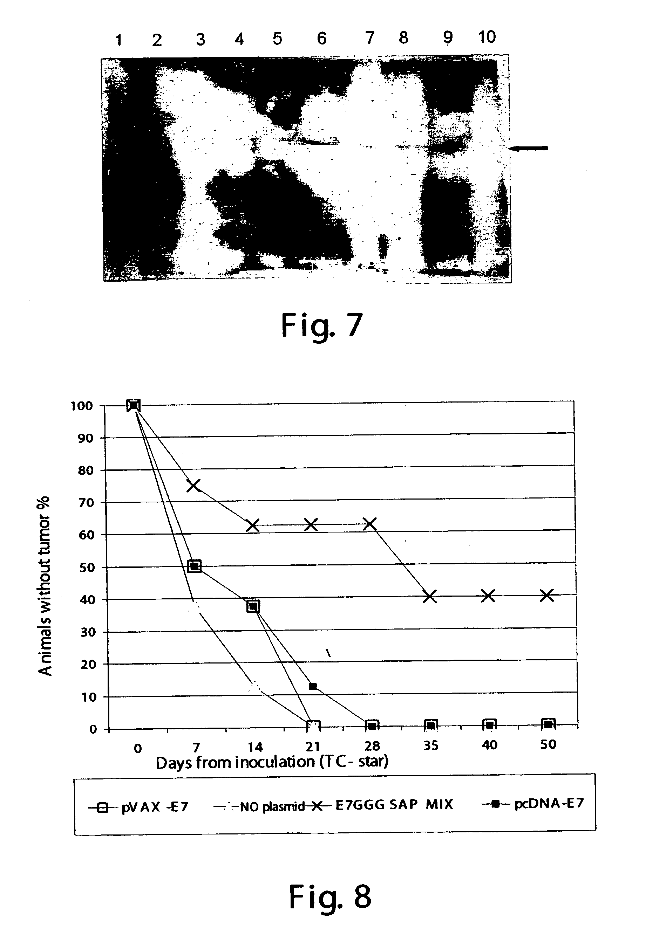

In parallel, various gene fusions have been cloned in a vector for plant transiently expression (Fig. 6). Preliminary experiments prove the expression of the SAP-KQ-E7GGG fusion protein (Fig. 7) and version thereof provided with signal sequence deriving from saporin (SAPss) and called ssSAP-KQ-E7GGG (not shown). These experiments are in progress for other constructs (eventually also with other expression vectors for successive purification).

Chimera vaccine ability to induce an immune response suitable to control the tumour growth has been evaluated in a murine model (C57Bl/6 mice). In this model, the vaccine preparations (specifically, a mixture of plasmids containing pVAX/E7GGG-SAP-KQ, pVAX/SAP-KQ-E7GGG, pVAX/ssSAP-KQ-E7GGG gene fusions) have been proved to be suitable to block the growth of experimental tumours expressing HPV16 E7 oncoprotein. It is to be pointed out that, in the used therapeutic immunization preclinical model (TC-1 star cells), the tumour is particularly aggressive and resistant to activity of other vaccine preparations which had shown effectiveness in other experimental models (DNA-based vaccines expressing unique E7 protein). In spite of this greater aggressiveness of TC-1 star cell induced tumour, the chimera vaccines have displayed an higher activity than other DNA-based preparation in blocking and/or delaying the tumour growth, demonstrating their effectiveness as vaccine in a clear way (Fig. 8). In conclusion the chimera vaccines which are the object of the present invention are biologically active in blocking the growth of an already implanted tumour.

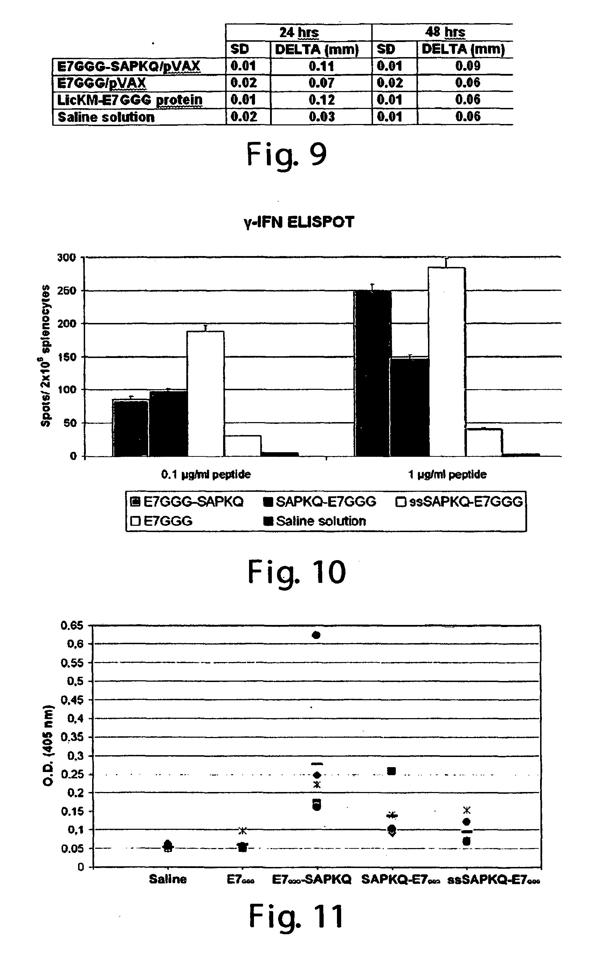

In order to study more extensively the ability of SAP-KQ carrier to activate a cell-mediated E7-specific response (mainly associated with the protection against the development of HPV oncologic lesions), the 'Delayed Type Hypersensitivity Response' (DTH, considered as an index of inflammatory antigen-specific Th1 type cytokine-mediated response) against E7 protein in plasmid pVAX/E7GGG-SAP-KQ vaccinated mouse has been evaluated in order to obtain a concept test. A positive, E7 protein specific response, higher than induced by pVAX/E7GGG plasmid immunization, and with peak 24 hours after the immunization has been firstly demonstrated for pVAX/E7GGG-SAP-KQ plasmid (ear thickening equal to 11 x 10⁻² ± 1 mm) (Figure 9).

Successively, by means of IFN-gamma ELISPOT, the contribution of individual plasmids to induction of cell-mediated type immune response has been evaluated. Individual plasmids have been administered in preclinical model without tumour cell 'challenge'. All individual plasmids displayed higher ability than single E7 gene in inducing the selection of E7-specific lymphocyte cytotoxic type clones as demonstrated by ELISPOT analysis carried out on immunized mice splenocytes and the ability of the mixture to block the TC-1 star tumour growth is presumably derived from. Among all, pVAX/ssSAP-KQ-E7GGG plasmid has demonstrated highest immuno-stimulating' activity inducing the greatest number of 'IFN-gamma secreting' splenocytes (Fig. 10).

Further, sera from pVAX/E7GGG-SAP-KQ, pVAX/SAP-KQ-E7GGG, pVAX/ssSAP-KQ-E7GGG plasmid immunized mice are reactive against E. coli produced HPV16 E7 recombinant protein and in higher extent with respect to pVAX/E7GGG (Fig. 11). Chimera plasmid immunization is, therefore, also suitable to specifically stimulate the immune branch of immune system against the tumour antigen (in this case HPV16 E7).

Anti-HPV therapeutic vaccines according to the present invention, in addition to treatment of pre-invasive and invasive oncologic lesions of uterine cervix whether in parallel to surgical or chemotherapeutic treatments in affected subjects, can be employed for the treatment of other high risk HPV pathologies; in particular against 50% of tonsil carcinomas, 40% of penis cancer and 25% of head-neck cancer. These vaccines can be employed for therapy of lesions preceding neoplastic degeneration for cervical cancer, like type 2 or 3 cervical intraepithelial neoplasias (CIN) in already affected or age reason prophylaxis program excluded women. Such treatment would not involve all the feminine population (as, on the contrary, prophylactic vaccination) with remarkable cost reduction to be charged to National Sanitary System.

E7GGG-SAP-KQ genetic chimeras, as genetic or protein vaccines expressed in plant using available technologies (stable or transient), or in other heterologous expression systems can be used for the production of new therapeutic vaccines suitable to stimulate the immune system of HR-HPV affected patients and enhancement of cytotoxic, mainly cytotoxic T type, antiviral response. The use of SAP-KQ as a new 'carrier' molecule is applicable also for other pathologies, especially those wherein, due to pathogenic antigen induced immunotolerance, is of crucial importance to generate a pro-inflammatory environment suitable to elicit an appropriate immune response.

It is therefore a specific object of the present invention a chimera sequence comprising or consisting of at least a nucleotide sequence encoding for a mutagenically non-oncogenic made tumoral human papilloma virus antigen (HPV) and a nucleotide sequence encoding for a mutagenically non-toxic made ribosome inhibiting protein (RIP), wherein said at least a sequence encoding for a tumoral human papilloma virus antigen is fused at 3' and/or 5' end of said sequence encoding for a ribosome inhibiting protein.

Particularly, the sequence encoding for a tumoral human papilloma virus antigen (HPV) can be selected from those encoding for E7, E6, E2, E5, L2, L1, preferably E7 and E6, HPV16 and HPV18 or fragments thereof. Meanwhile, the sequence encoding for a ribosome inhibiting protein (RIP) can be chosen from those encoding for type 1 RIP, such as for example saporin, gelonin, tricosanthin, luffin, PAP, preferably saporin, double-chain RIP A chain or other type 2 RIP, such as for example, abrin, ebulin, or fungal type RIP, such as for example clavin or fragments thereof.

Still more preferably, the sequence encoding for a tumoral human papilloma virus antigen (HPV) can be mutated at retinoblastoma binding site. The sequence encoding for a tumoral human papilloma virus antigen can be E7 gene wherein the nucleotide sequence
nt 61 GAT CTC TAC TGT TAT GAG (SEQ ID NO:3)
encoding for DLYCYE (SEQ ID NO: 4) motif is mutated in nucleotide sequence
nt 61 GGT CTC TAC GGT TAT GGG (SEQ ID NO:5)
encoding for GLYGYG (SEQ ID NO: 6) motif.
where D= Aspartic Acid, G = Glycine, C = Cysteine, E = Glutamic Acid.

The sequence encoding for a ribosome inhibiting protein (RIP), whether saporin or other type 1 RIP or double-chain RIP A chain or also a fungal origin RIP as for example clavin, can be mutated in such a way that catalytic activity loss results, as for example by means of substitution of catalytic site invariant amino acids, as for example glutamic acid and arginine, substituted by lysine and glutamine, respectively.

In the case of saporin the wild type nucleotide sequence:
nt 512 ATT CAG ATG ACG GCT GAG GCA GCG CGA TTT AGG TAC (SEQ ID NO:7)
encoding for:
aa 171 I Q M T A E A A R FRY (SEQ ID NO:8) motif
is muatetd in nucleotide sequence
nt 512 ATT CAG ATG ACG GCT AAG GCA GCG CAG TTT AGG TAC (SEQ ID NO:9)
encoding for:
aa 171 I Q M T A K A A Q FRY (SEQ ID NO:10) motif
where E= Glutamic Acid K = Lysine, R = Arginine, Q = Glutamine

The chimera sequence according to the present invention can further comprises at least a nucleotide sequence encoding for a signal peptide located at 5' end of said chimera sequence. In particular, the signal peptide can be a signal peptide for secretion as for example the signal peptide for directing to apoplastic SAP (SAPss) endoplasmic reticulum, or a 3' peptide of said sequence, as for example for 'targeting' in determined sub-cellular compartments (as for example KDEL Tetrapeptide for endoplasmic reticulum).

Vector comprising chimera sequence as above defined or host cell comprising said vector, as for example mammalian, insect, vegetal, yeast, unicellular alga o bacterial cell are a further object of the present invention.

The present invention further refers to a chimera protein encoded by the chimera sequence as above defined.

A further aspect of the present invention concerns a vaccine comprising or consisting of as above defined chimera sequence, or comprising or consisting of as above defined vector or comprising or consisting of as above defined chimera protein.

The use of as above defined chimera sequence or as above defined vector or as above defined chimera protein for the preparation of a medicament for the treatment and prevention of HPV deriving infections and/or complications, as for example tonsil carcinoma, penis cancer, head-neck cancer, uterus cancer, intraepithelial cervical neoplasia.

The present invention further concerns the use of ribosome inhibiting protein (RIP), as for example type 1 RIP, as for example saporin, gelonin, tricosanthin, luffin, PAP, preferably saporin, double-chain RIP A chain as for example ricin or other type 2 RIP, such as for example, abrin, ebulin, or fungal type RIP, said protein being made not toxic by mutagenesis and being in combination with an antigen, as for example papilloma human virus tumoral antigen or other viruses as SARS, Infuence, Hepatitis, as adjuvant in order to enhance said antigen immunogenicity. Preferably, the ribosome inhibiting protein (RIP) is saporin mutated in such way that glutamic acid-176 and arginine-179 amino acids in primary sequence are replaced by lysine and glutamine, respectively. Particularly, human papilloma virus (HPV) tumoral antigen can be selected from HPV-16 or HPV-18 E7, E6, L2, L1, E2, E5 or fusions thereof with tumoral antigens. E7 human papilloma virus (HPV) tumoral antigen can be mutated at retinoblastoma binding site, in particular E7 gene can be mutated as above described.

The present invention now will be described by an illustrative, but not limitative way, according to preferred embodiments thereof, with particular reference to enclosed drawings wherein:
Figure 1 shows some examples of several partial amino acid sequences of RIP catalytic site (SEQ ID NO: 11, SEQ ID NO: 12, SEQ ID NO: 13).
Figure 2 shows a schematic representation of apoSAP-KQ mutant. Dark gray bars represent mature saporin and is preceded by a signal peptide (white) and followed by C-terminal pro-peptide (light gray) where a putative glycosylation signal (NST) is present; the catalytic site region is boxed and two changed amino acids are in bold letters.
*Figure 3* *(a and b) shows a scheme of SAP-KQ and ssSAP-KQ genes and SAP-KQ with E7GGG gene fusions, in various orientations, with or without secretion signals. Expression and purification results for SAP-KQ and ssSAP-KQ proteins (a) and fusion expression results (b)* in E. coli *cells are shown. Genes have been inserted in pQE-30 vector by directional cloning of* *Sph and Sal I fragments.*
Figure 4 shows a schematic representation of pVAX constructs (single or fused) in view of HEK-293 transfection and immunization experiments. Genes have been inserted by directional cloning as *Pst* and *Xho* fragments under the control of Citomegalovirus promoter in pVAX plasmid.
Figure 5 shows by an exemplificative way, an expression of E7GGG-SAP-KQ chimera fusions in mammalian cells. pVAX/E7GGG, pVAX/SAP-KQ and pVAX/E7GGG-SAP-KQ plasmids have been used for transient expression and transfection in HEK 293 mammalian cells. Cellular lysates have been obtained as a result of transfection in the presence of proteasome MG-132 (25 µM) inhibitor. Proteins have been separated by SDS-PAGE. The presence of expected proteins has been determined by means of immunoblotting using anti-Saporin (A panel) or anti-E7 (B panel) polyclonal antibodies. A panel: 1 line : molecular weight marker; 2 line: pVAX/SAP-KQ (approximately 28 K); 3 and 4 lines: pVAX/E7GGG-SAP-KQ (- 50 K). B panel: 1 line: pVAX/E7GGG (approximately 17 K, theoretical weight 11 K); 2 line: molecular weight marker; 3 line: pVAX/E7GGG-SAP-KQ (∼ 50 K)
Figure 6 shows a schematic representation of constructs (single or fused) used for transfection preliminary experiment of *Nicotiana benthamiana* plants. Genes have been inserted by directional cloning in pPVX-201 plasmid. In pPVX-201 vector, deriving from PVX genome, the CaMV 35S term refers to 35S promoter of cauliflower mosaic virus; RdRp refers to the gene encoding for protein needed for PVX replication; M1, M2, M3 indicate three partially overlapped "open reading framers", encoding for 25, 12 and 8 KDa PVX proteins, respectively; the term CP means the gene for PVX enclosing protein; the term CPP indicates the gene duplicated promotor for enclosing protein; the NOS-term indicates transcription termination signal derived from *Agrobacterium tumefaciens* nopaline synthase gene.
Figure 7 shows the result of immunoblotting assay (Western blot) PVX-201- SAP-KQ E7GGG infected *Nicotiana benthamiana* plant extracted proteins (7 days after inoculation).
Figure 8 shows therapeutic vaccination data for mouse in which a particularly aggressive, E7 antigen expressing experimental tumour was already present. Chimera vaccines displayed higher activity in delaying and inhibiting the tumour growth.
Figure 9 shows data relating to cell-mediated response as evaluated by means of 'Delayed Type Hypersensitivity' for pVAX-E7GGG-SAP-KQ construct.
Figure 10 shows data relating to cell-mediated response as evaluated by means of ELISPOT assay for all the constructs.
Figure 11 shows the reactivity of sera from mouse vaccinated with different construct against E7 HPV16 protein (vaccination humoral response).

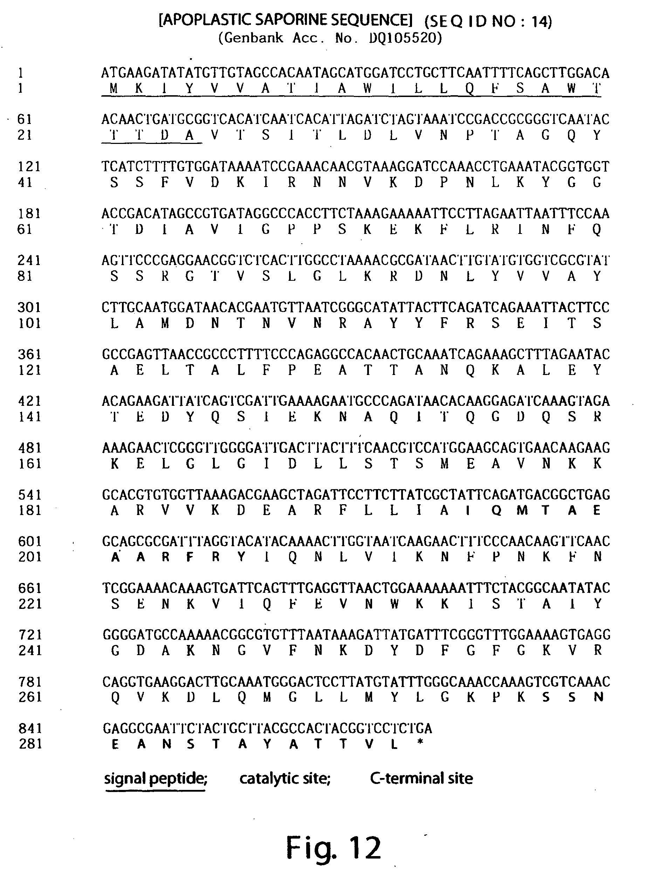

Figure 12 shows apoplastic saporin sequence (SEQ ID NO: 14)

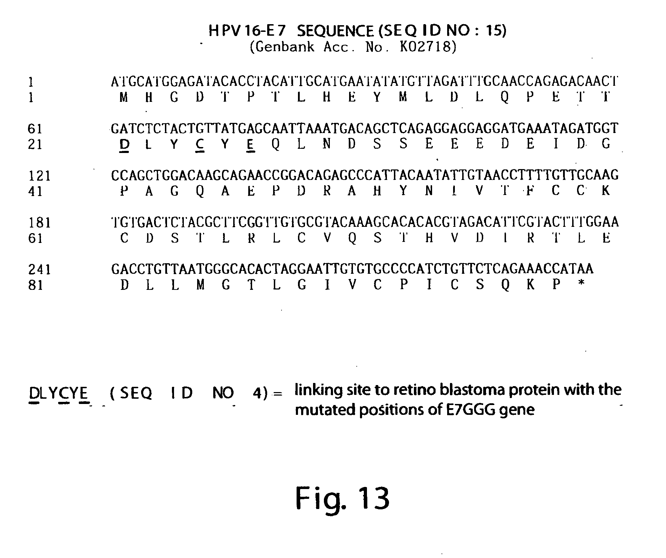

Figure 13 shows HPV16-E7 sequence (SEQ ID NO: 15) **EXAMPLE 1:** *Effectiveness study of the vaccine according to the invention*

### MATERIALS AND METHODS

### Animals and cell lines

C57BL/6 female 6 week mouse (Charles River - Como, Italy) have been maintained in specific pathogenic free conditions at the Istituto Tumori Regina Elena stabulary (Rome, Italy). All the experiments have been carried out according to institutional guidelines for laboratory animals use.

E7 expressing TC-1 cells were a kind gift by Prof. T.C. Wu (Johns Hopkins Medical Institutions, Baltimore, MD). Particularly aggressive TC-1 star clone was obtained by experimental animal repeating passages (Venuti et *al.,* 2009).

HEK-293 cells (Human Embryo Kidney) used for transfection experiments were a kind gift by Prof. D. Neri (ETH-Hönggerberg, Zurich, Switzerland).

### HPV-16 E7 (E7GGG), SAP (SAP-KQ) gene mutagenesis and production of recombinant vectors for expression in mammalian cells.

In order to overcome the problems associated to the clinical use of oncogenes and toxic protein expressing genes, attenuated HPV-16 E7 and SAP genes, without transforming or N-glycosylation activity, have been used.

In particular, DLYCYE motif (amino acid 21-26) (SEQ ID NO: 4) of HPV-16 E7 gene has been mutated to GLYGYG sequence (SEQ ID NO: 6), obtaining E7GGG gene (Smahel et *al*., 2001). HPV-16 E7 oncogene (CoreNucleotide accession number K02718), cloned in bacterial expression vector pQE30, has been mutated using "QuikChange site-directed mutagenesis kit" (Stratagene, La Jolla, CA). E7GGGfor (5'-CAACAAGAGA-CAACTGGTCTCTACGGTTATGGGCAATTAAATGACAGC-3") (SEQ ID NO:16) and E7GGGrev (5"-GCTGTCATTTAATTGCCCATAACCGTAGA-GACCAGTTGTCTCTGGTTGC-3") (SEQ ID NO:17) primers, are used in order to insert 3 point mutations characterizing 'E7GGG' attenuated gene in Retinoblastoma (pRb) protein binding site, indicated in bold letters. Inserted mutations result in the substitution of 3 amino acids in HPV-16 E7 protein sequence: D₂₁G (Aspartic Acid₂₁ > Glycine), C₂₄G (Cysteine₂₄ > Glycine) and E₂₆G (Glutamic Acid₂₆ > Glycine). The sequence authenticity has been confirmed by pQE30/E7GGG construct sequencing used in order to transform XL1-Blue *Escherichia coli* competent cells.

IQMTAEAARFRY motif (SEQ ID NO: 8) of *Saponaria officinalis* apoplastic saporin encoding gene (amino acids 195-206, corresponding to catalytic site of SAP named gene, Genbank Acc. No. DQ105520) has been mutated to IQMTAKAAQFRY sequence (SEQ ID NO: 10), obtaining SAP-KQ gene, according to methodology already described for HPV-16 E7. *SapoA For mut KQ* (5'-CT ATT CAG ATG ACG GCT **A**AG GCA GCG C**AG** TTT AGG TAC ATA C-3') (SEQ ID NO:18) and *SapoA Rev mut KQ* (3'-G TAT GTA CCT AAA CTG CGC TGC CTT AGC CGT CAT CTG AAT AGC-3') (SEQ ID NO:19), have been used in order to insert point mutations characterizing 'SAP-KQ' attenuated gene indicated in bold letter. Inserted mutations result in the substitution of 2 amino acids in *Saponaria officinalis:* apoplastic saporin: E₁₇₆K (Glutamic Acid₁₇₆ > Lysine) and R₁₇₉Q (Arginine₁₇₉ > Glutamine). Sequence authenticity has been confirmed by pQE-30/SAP-KQ construct sequencing used in order to transform XL1-Blue *Escherichia coli* competent cells.

E7GGG and SAP-KQ genes as well as His6-E7GGG and His6-SAP-KQ have been expressed in E. *coli* and have been purified both in denaturing and not denaturing conditions. The preparation purity has been verified on 12 % SDS-PAGE (sodium dodecyl sulphate-polyacrylamide gel) by Coomassie blue staining and immunoblotting using a policlonal antibody specific for said proteins, respectively (produced in laboratory using immunized animals, 1:1000 dilution), biotinylated anti-rat antibody (1:5000 diluted) for E7 and biotinylated anti-rabbit antibody (1:5000 diluted) for SAP-KQ, and streptavidin peroxidase conjugated (1:2000 diluted), supplied by GE Healthcare Life Sciences (Uppsala, Sweden). Antibody specific binding has been evaluated by means of chemiluminescence (ECL Plus system, GE Healthcare Life Sciences).

In order to carry out the cloning in pVAX vector for the expression in mammalian cells (Invitrogen, Carlsbad, CA), E7GGG gene has been amplified using E7CPSFor (5' -G GCC atc gat CTG CAG GCATGC ATC *ATG* CAT GGA GAT ACA CCT ACA TT-3') (SEQ ID NO:20) primer, wherein PstI site is underlined, ClaI site is in small letters, Kozac sequence for the regulation of mammalian cell expression is in bold letters and triplet for translation initiation is in italics and E7XSRev (5'-G GCC CTC GAG gtc gac *TTA* TGG TTT CTG AGA ACA GAT GG-3') (SEQ ID NO: 21) primer, wherein XhoI restriction site is underlined, SalI is small letters and stop triplet is in italics.

SAP-KQ gene has been amplified using *SapoA CPS For* (5'-G GCC atc gat CTG CAG GCA TGC **A**TC *ATG* GTC ACA TCA ATC ACA TTA GAT C- 3') (SEQ ID NO:22) primer, wherein PstI site is underlined, ClaI site is in small letters, Kozac sequence for the regulation of mammalian cell expression is in bold letters and triplet for translation initiation is in italics and E7XSRev (5'-G GCC CTC GAG gtc gac *TTA* TGG TTT CTG AGA ACA GAT GG-3') (SEQ ID NO:21) primer, wherein XhoI restriction site is underlined, SalI is in small letters and stop triplet is in italics. After restriction using PstI and XhoI enzymes, resultant PCR products have been cloned in pVAX vector obtaining pVAX/E7GGG and pVAX/SAP-KQ constructs.

E7GGG gene has been fused 'in frame' directly upstream or downstream of SAP-KQ gene lacking of relative secretion pathway directing sequence (nucleotides 1-72) obtaining E7GGG-SAP-KQ or SAP-KQ-E7GGG sequences, respectively. Genes have been fused by means of SOE-PCR ('splicing by overlap extension-polymerase chain reaction'). For E7GGG-SAP-KQ fusion, E7GGG gene has been amplified using the following primers: *E7CPSFor* (SEQ ID NO: 20) and *SapoA E7 Rev* (5'-TAA TGT GAT TGA TGT GAC TGG TTT CTG AGA ACA GAT GGG-3') (SEQ ID NO: 24) incorporating 5' region of SAP gene lacking of relative secretion pathway directing sequence and 3' nucleotides of E7 gene. For the amplification of SAP-KQ gene for fusion purpose, E7 *SapoA For* (5'-ATC TGT TCT CAG AAA CCA GTC ACA TCA ATC ACA TTA GAT C-3') (SEQ ID NO: 25) primer, incorporating E7 gene upstream of nucleotides and SAP-KQ gene initial region and *SapoA XS rev* primer (SEQ ID NO: 23) have been used.

In order to obtain SAP-KQ-E7GGG fusion, SAP-KQ gene has been amplified using *SapoA CPS For* (SEQ ID NO: 22) primer and E7 *SapoA Rev* (5'-TGT AGG TGT ATC TCC ATG CTT TGG TTT GCC CAA ATA CAT AAG-3') (SEQ ID NO: 26), incorporating 5' region of E7 gene and 3' nucleotides of SAP gene, primer. E7GGG gene has been amplified using *E7 XS Rev* (SEQ ID NO: 21) and *SapoA E7 For* (5'-TAT TTG GGC AAA CCA AAG CAT GGA GAT ACA CCT ACA TTG C-3') (SEQ ID NO: 27), incorporating SAP gene upstream of nucleotides and E7 gene initial region.

Also SAP-KQ gene, containing secretion pathway directing sequence, has been fused 'in frame' upstream of E7GGG gene (ssSAP-KQ-E7GGG). In this case, primers used for gene amplifications aiming to fusion have been: *SapoAss CPS For* (5'-G GCC ATC GAT CTG CAG GCATGC ATC ATG AAG ATA TAT GTT GTA GCC AC-3') (SEQ ID NO: 28) and E7 *SapoA Rev* (SEQ ID NO: 26) for SAP-KQ gene, and *E7XSrev*/*SapoA E7 For* (SEQ ID NO: 21/SEQ ID NO: 27) pair for E7GGG gene.

Assembled genes, have been cloned in pBluesScript-SK(+) vector, sequenced and then subcloned in pVAX for mammalian cell expression after restriction with the PstI and XhoI enzymes obtaining pVAX/E7GGG-SAP-KQ, pVAX/SAP-KQ-E7GGG and pVAX/ssSAP-KQ-E7GGG constructs. All the plasmids have been disseminated in *E. coli* XL1-Blue.

Plasmids used for immunization experiments have been extracted by means of alkaline lysis followed by purification on CsCl gradient. Plasmids used for transfection experiments have been prepared using 'EndoFree plasmid maxi kit' (Qiagen, Hilden, Germany).

### Transfection of mammalian cells

HEK-293 cells grown in 6-well cell culture plates, have been transfected (Effectene transfection kit; Qiagen) the following day with 1 µg of each plasmid, respectively. Expression of E7GGG, E7GGG-SAP-KQ and SAP-KQ-E7GGG proteins in secretory and not secretory forms has been assayed by immunoblotting as already described for His6-E7GGG or His6-SAP-KQ.

### Immunoblotting evaluation of fusion product expression after transfection.

The expression of fusion proteins has been analyzed by immunoblotting after transient transfection of approximately 1 x 10⁶ cells of HEK-293 cell line in the presence of MG-132 (carbobenzoxy-L-leucyl-L-leucyl-L-leucinal in dimethyl sulfoxide [DMSO]; Calbiochem, Darmstadt, Germany) proteosome inhibitor added to culture medium 24 hours after the transfection. 48 hours after transfection, the cells have been washed twice with cold PBS, detached using a 'scraper', resuspended in 'SDS loading buffer' 5X with betamercaptoethanol diluted to a 1x sample concentration and boiled over 10 minutes. The expression analysis of proteins separated on SDS-PAGE and transferred on polyvinylidene fluoride (PVDF) membrane has been carried out as already described for His6-E7GGG or His6-SAP-KQ.

### Challenge of animals with E7 expressing experimental tumours and genetic vaccination.

Before the vaccination with different E7GGG and SAP-KQ containing plasmids, groups of 8 mouse have been inoculated subcutaneously in the flank with a tumorigenic dose of HPV-16 E7 protein expressing TC-1 star cells (5x10⁴ cells). After the immunization, the tumour growth has been evaluated two times weekly by means of visual and tactile inspection. A group of mouse has not immunized as a control of natural growth of TC-1 induced tumour. The mouse have been immunized by intramuscular inoculum of 100 µg of DNA from several vaccine preparations at 3 and 10 days after tumour inoculum. Immunization negative control group has been administered with sterile saline.

### Production of recombinant vectors for plant expression.

In order to carry out the chimera gene cloning in PVX-derived vector, pPVX-201, for transient plant expression, E7GGG-SAP-KQ SAP-KQ-E7GGG and ssSAP-KQ-E7GGG assemblies (obtained as described in paragraph concerning the production of constructs for mammalian expression) have been cloned in pPVX-201 vector after restriction with ClaI and SalI enzymes obtaining pPVX-201/E7GGG-SAP-KQ, pPVX-201/SAP-KQ-E7GGG and pPVX-201/ssSAP-KQ-E7GGG constructs. All the plasmids have been propagated in *E. coli* XL1-Blue.

### Plant infection and immunoblotting

Plasmids used for plant transient expression have been prepared using 'Plasmid maxi kit' (Qiagen, Hilden, Germany). Two leaves from four leave stage, containment greenhouse grown *Nicotiana benthamiana* plant (biosafety level 2) have been infected by mechanical abrasion with 10 µg of different pPVX-201/E7GGG-SAP-KQ, pPVX-201/SAP-KQ-E7GGG and pPVX-201/ssSAP-KQ-E7GGG named plasmids. Insert free, "wild type' pPVX plasmid has been used as control. Both inoculated and systemic leaves have been collected at infection symptoms occurrence and stored at -80°C. Frozen vegetal material has been immersed in liquid nitrogen containing mortar, finely powdered using a pestle, re-suspended in phosphate buffer (PBS) and centrifuged at 11000 rpm over 30 minutes (Franconi et *al.,* 2002). Total soluble protein content (TSP) in supernatant obtained from vegetal material has been determined by means of Bradford assay (BioRad, Hercules, CA, USA) using bovine serum albumin as standard. Approximately 20 µg of TSP have been separated by 12% SDS-PAGE, transferred on Immobilon-P (Millipore, Bedford, But, USA) membrane and blocked with PBS containing 5% milk. Immunoblotting analysis has been carried out as already described for His6-E7GGG or His6-SAP-KQ.

### Evaluation of cell-mediated response

CTL response has been assayed by ELISPOT according to Miyahira, et *al.* (1995). Briefly: 96-well filtration plates have been covered with mice anti-interferon gamma (IFN γ) antibodies and incubated overnight, in order to capture activated T-cell secreted IFN γ. In order cell precursors CD4 and CD8 to be detected, splenocytes from vaccinated mouse have been collected a week after the booster and added to wells together with interleukyne-2 (15 unit/ml). The cells have been incubated over 24 hours with or without peptides representing specific E7 (H-2D^{b}) CTL (aa. 30-67 and aa. 49-57) epitopes. After the culture, the plates have been washed, incubated with biotinylated anti-IFN-γ mice antibodies, again washed and incubated with avidin conjugated alkaline phosphatase. IFN-γ-antibody reaction is detected by "spot", evidenced by BCIP/NBT.

### Determination of E7-specific humoral response

The presence of E7-specific IgG in sera from various plasmid immunized mouse has been assayed by ELISA. Polystyrene 96-well plates have been covered and incubated overnight at 4°C with phosphate buffer diluted His-E7 protein (1 µg/ml).

After stop with 5 % skimmed milk containing PBS solution for 2 hours, sera from immunized mouse diluted at 1:50 or 1:100 in 0.5% Tween-20 containing PBS buffer, have been added to the samples. Total IgGs has been detected by biotin conjugated goat anti-mice antibodies. Signal has been amplified by adding horseradish peroxidase conjugated streptavidin. The reaction has been developed by addition of ABTS (2,2-azo-bis-benzthiazoline-6-sulphonic acid) solution. Sample optical density has been determined at 405 nm using an ELISA reader.

### E7 protein delayed hypersensitivity (DTH).

E7 protein DTH has been evaluated according to technique as described by Dunn et *al.* (1997). Shortly, 5 µg of PBS diluted His-E7 protein have been injected intradermally into an ear of vaccinated mouse in order to control the inflammatory response to protein preparation. Ear thickness has been determined 48 and 96 hours after His-E7 inoculation using a microgauge. The other ear of same mouse has been inoculated only with PBS, as a control. Ear thickness increase resulting from delayed hypersensitivity response has been calculated as thickness difference of His-E7 inoculated and control ear.

### REFERENCES

- Bergamaschi G., Perfetti V., Tonon L., Novella A., Lucetti C., Da nova M., Glennie M.J., Merlini G., Cazzola M. (1996). Saporin, a ribosome-inactivating protein used to prepare immunotoxins, induces cell death via apoptosis. British Journal of Haematology. 93: 789-794.
- Bonini F., Traini R., Comper F., Fracasso G., Tomazzolli R, Dalla Serra M., Colombatti M. (2006). N-terminal deletion affects catalytic activity of saporin toxin. J Cell Biochem. 98: 1130-9.
- Cheung L.H., Marks J.W., Rosenblum M., (2004). Development of 'designer toxins' with reduced antigenicity and size. Proc. Am. Assoc. Cancer Res. 45: 874-875.
- Dunn L.A., Evander M., Tindle R.W., Bulloch A.L., de Kluyver R.L., Fernando G.J., Lambert P.F., Frazer I.H. (1997). Presentation of the HPV16E7 protein by skin grafts is insufficient to allow graft rejection in an E7-primed animal. Virology. 235: 94-103.
- Ferreras J.M., Barbieri L., Girbes T., Battelli M.G., Rojo M.A., Arias F.J., Rocher M.A., Soriano F., Mendez E., Stipre F. (1993). Distribution and properties of major ribosome inactivating proteins (28S rRNA N- glycosidases) of the plant Saponaria officinalis L. (Caryophyllaceae) Biochim. Biophys. Acta 1216: 31-42.
- Franconi R., Di Bonito P., Dibello F., Accardi L., Muller A., Cirilli A, Simeone P., Dona G., Venuti A., Giorgi C. (2002). 'Plant-derived Human Papillomavirus 16 E7 oncoprotein induces immune response and specific tumor protection'. Cancer Research 62: 3654-58.
- Franconi R. & Venuti A. (2006). 'HPV Vaccines in Plants: an appetising solution to Control Infection and Associated Cancers' in 'Papillomavirus Research: from Natural History to Vaccines and Beyond'. Edited by M. Saveria Campo, Caister Academic Press, Norfolk, NR18 0JA, U.K. pp. 357-372.
- Franconi R., Massa S., Illiano E., Muller A., Cirilli A., Accardi L., Di Bonito P., Giorgi C., Venuti A. (2006). Exploiting the plant secretory pathway to improve the anti-cancer activity of a plant-based HPV16 E7 vaccine. The International Journal of Immunopathology and Pharmacology 19: 187-197.
- Frankel A., Welsh P., Richardson J., Robertus J.D. (1990). Role of arginine 180 and glutamic acid 177 of ricin toxin A chain in enzymatic inactivation of ribosomes. Mol. and Cell. Biol. 10(12): 6257-6263.
- Frankel A.E. (2004). Reducing the Immune Response to Immunotoxin, Clinical Cancer Research 10: 13-15.
- Frazer I.H. (2004). Prevention of cervical cancer through papillomavirus vaccination. Nat. Rev. Immunol. 4: 46-54.
- Hartley M.R, & Lord J.M. (2004). Cytotoxic ribosome-inactivating lectins from plants. Biochim Biophys Acta 1701: 1-14.
- Hur Y., Hwang D.J., Zoubenko O., Coetzer C., Uckun F.M., Turner N.E. (1995). Isolation and characterization of pokeweed antiviral protein mutations in Saccharomyces cerevisiae: identification of residues important for toxicity. Proc. Natl. Acad. Sci. USA 92: 8448-8452.
- Ma B., Xu Y., Hung C., Wu T.C., (2010). HPV and therapeutic vaccines: where are we in 2010? Curr. Cancer Ther. Rev. 6: 81-103.
- Marshall R.S., D'Avila F., Di Cola A., Traini R., Solinas M., Spanò L., Fabbrini M.S., Ceriotti A. (2010). Signal peptide regulated toxicity of a plant ribosome-inactivating protein during cell stress(submitted)
- Massa S., Franconi R., Brandi R., Muller A., Mett V., Yusibov V., Venuti V. (2007). Anti-cancer activity of plant-produced HPV16 E7 vaccine. Vaccine 25: 3018-3021.
- Massa S., Simeone P., Muller A., Benvenuto E., Venuti A., Franconi R. (2008). Cancer immunotherapy by DNA vaccines expressing the Human Papillomavirus 16 E7 protein fused to a plant virus coat protein. Human Gene Therapy 19: 354-64.
- Miyahira Y., Murata K., Rodriguez D., Rodriguez J.R., Esteban M., Rodrigues M.M., Zavala F. (1995). Quantification of antigen specific CD8+ T cells using an ELISPOT assay. J. Immunol. Met. 181: 45-54.
- Ng T.B., Wong J.H., Wang H. (2010). Recent progress in research on ribosome inactivating proteins Curr. Prot. Pept. Sci. 11: 37- 53.
- Pittaluga E., Tucci A., Poma A., Spanò L. (2005). Expression and characterization in E. coli of mutant forms of saporin. J. Biotechnology 117: 263-266.
- Santanchè S., Bellelli A., Brunori M. (1997). The unusual stability of saporin, a candidate for the synthesis of immunotoxins. Biochem Biophys Res. Commun. 234 (1): 129-132.
- Smahel M, Sima P, Ludvíková V, Vonka V. (2001). Modified HPV16 E7 Genes as DNA Vaccine against E7-Containing Oncogenic Cells. Virology. 281(2):231-8**.**
- Szalai K., Scholl I., Forster-Waldl E., Polito L., Bolognesi A., Untersmayr E., Riemer A.B., Boltz-Nitulescu G., Stirpe F., Jensen-Jarolim E. (2005). Occupational sensitization to ribosome-inactivating proteins in researchers. Clin Exp Allergy 35: 1354-1360
- Stirpe, F. (2004). Ribosome inactivating proteins. Toxicon 44: 371-383.
- Stirpe, F., Battelli, M.G. (2006). Ribosome-inactivating proteins: progress and problems. Cellular and Molecular Life Sciences, 63:1850-1866.
- Tartarini A., Pittaluga E., Marcozzi G., Testone G., Rodrigues-Pousada R.A., Giannino D., Spanò L. (2010). Differential expression of saporin genes upon wounding, ABA treatment and leaf development. Physiol. Plant. in press
- Venuti A, Massa S, Mett V, Vedova L.D., Paolini F, Franconi R, Yusibov V. (2009). An E7-based therapeutic vaccine protects mice against HPV16 associated cancer. Vaccine. 27:3395-7.
- Zarovni N., Vago R., Soldà T., Monaco L., Fabbrini M.S. (2007). Saporin as a novel suicide gene in anticancer gene therapy. Cancer Gene Ther. 14:165-73.
- Zhao J., Ben L-H., Wu Y-L., Hu W., Ling K., Xin S-M., Nie H-L., Ma L., Pei G. (1999). Anti-HIV Agent Trichosanthin Enhances the Capabilities of Chemokines to Stimulate Chemotaxis and G Protein Activation, and This Is Mediated through Interaction of Trichosanthin and Chemokine Receptors. J. Exp. Med. 190: 101-111.

## Claims

1. Chimera sequence comprising or consisting of at least a nucleotide sequence encoding for a mutagenically rendered non-oncogenic tumoral human papilloma virus antigen (HPV) and a nucleotide sequence encoding for a mutagenically rendered non-toxic ribosome inhibiting protein (RIP), wherein said at least a sequence encoding for a tumoral human papilloma virus antigen is fused at 3' and/or 5' end of said sequence encoding for a ribosome inhibiting protein, said ribosome inhibiting protein (RIP) being saporin.

2. Sequence according to claim 1, wherein the sequence encoding for a tumoral human papilloma virus antigen is selected from those encoding for E7, E6, E2, E5, L2, L1, preferably E7 and E6, HPV16 and HPV18, or fragments thereof.

3. Sequence according to anyone of preceding claims, wherein the sequence encoding for a tumoral human papilloma virus antigen (HPV) is mutated at retinoblastoma binding site.

4. Sequence according to claim 3, wherein the sequence encoding for a mutated tumoral human papilloma virus antigen is E7 gene wherein DLYCYE motif (SEQ ID NO:4) has been mutated in GLYGYG sequence (SEQ ID NO:6).

5. Sequence according to anyone of preceding claims, wherein the sequence encoding for saporin is mutated in catalytic site invariant amino acids in such a way that catalytic activity loss results.

6. Sequence according to claim 5, wherein saporin 176-glutamic acid and 179-arginine amino acids are mutated to lysine and glutamine, respectively.

7. Sequence according to anyone of preceding claims, further comprising at least a nucleotide sequence encoding for a signal peptide located at 5' end of said chimera sequence.

8. Sequence according to claim 7, wherein the signal peptide is selected from the group consisting of a signal peptide directing to apoplastic SAP endoplasmic reticulum (SAPss) or for the secretion of other plant proteins.

9. Vector comprising the sequence as defined according to anyone of preceding claims.

10. Host cell comprising the vector as defined according to claim 9.

11. Chimera protein encoded by the chimera sequence as defined according to anyone of claims 1 to 8.

12. Vaccine comprising or consisting of the chimera sequence as defined in anyone of claims 1 to 8, or comprising or consisting of the vector as defined according to claim 9 or comprising or consisting of the chimera protein as defined according to claim 11.

13. Chimera sequence as defined according to anyone of claims 1 to 8 or vector as defined according to claim 9 or chimera protein as defined according to claim 11 for use in the treatment and prevention of HPV deriving infections and/or complications.

14. Chimera sequence as defined according to anyone of claims 1 to 8 or vector as defined according to claim 9 or chimera protein as defined according to claim 11 for use according to claim 13, wherein HPV deriving infections and/or complications are selected from the group consisting of tonsil carcinoma, penis cancer, head-neck cancer, uterus cancer, intraepithelial cervical neoplasia.

15. Mutagenically rendered non-toxic ribosome inhibiting protein (RIP) in association with an antigen for use in the adjuvant treatment for increasing the immunogenicity of said antigen, wherein said ribosome inhibiting protein (RIP) is saporin.

## Patentansprüche

1. Chimäre Sequenz, welche mindestens eine Nukleotidsequenz, die für ein tumorales Human-Papillomavirus-Antigen (HPV), welches durch Mutagenese nicht-onkogen gemacht wurde, codiert, und eine Nukleotidsequenz, die für ein Ribosomen-inhibierendes Protein (RIP), welches durch Mutagenese nicht-toxisch gemacht wurde, codiert, umfasst oder daraus besteht, wobei die mindestens eine Sequenz, die für ein tumorales Human-Papillomavirus-Antigen codiert, an das 3'- und/oder 5'-Ende der Sequenz, die für ein Ribosomen-inhibierendes Protein codiert, fusioniert ist, und wobei das Ribosomen-inhibierende Protein (RIP) Saporin ist.

2. Sequenz nach Anspruch 1, wobei die Sequenz, die für ein tumorales Human-Papillomavirus-Antigen codiert, aus denjenigen, die für E7, E6, E2, E5, L2, L1, vorzugsweise E7 und E6, HPV16 und HPV18, codieren, oder Fragmenten davon ausgewählt ist.

3. Sequenz nach irgendeinem der vorhergehenden Ansprüche, wobei die Sequenz, die für ein tumorales Human-Papillomavirus-Antigen (HPV) codiert, an der Retinoblastom-Bindungsstelle mutiert ist.

4. Sequenz nach Anspruch 3, wobei die Sequenz, die für ein mutiertes tumorales Human-Papillomavirus-Antigen codiert, das E7-Gen ist, worin das Motiv DLYCYE (SEQ-ID-NO:4) zur Sequenz GLYGYG (SEQ-ID-NO:6) mutiert worden ist.

5. Sequenz nach irgendeinem der vorhergehenden Ansprüche, wobei die für Saporin codierende Sequenz an invarianten Aminosäuren der katalytischen Stelle so mutiert ist, dass ein Verlust der katalytischen Aktivität resultiert.

6. Sequenz nach Anspruch 5, wobei die Aminosäuren Glutaminsäure 176 und Arginin 179 von Saporin zu Lysin bzw. Glutamin mutiert sind.

7. Sequenz nach irgendeinem der vorhergehenden Ansprüche, welche ferner mindestens eine Nukleotidsequenz umfasst, die für ein Signalpeptid, lokalisiert am 5'-Ende der chimären Sequenz, codiert.

8. Sequenz nach Anspruch 7, wobei das Signalpeptid aus der Gruppe ausgewählt ist, die aus einem Signalpeptid, welches apoplastisches SAP zum endoplasmatischen Retikulum führt (SAPss), oder einem Signalpeptid zur Sekretion anderer Pflanzenproteine besteht.

9. Vektor, umfassend die Sequenz wie in irgendeinem der vorhergehenden Ansprüche definiert.

10. Wirtszelle, umfassend den Vektor wie in Anspruch 9 definiert.

11. Chimäres Protein, codiert von der chimären Sequenz wie in irgendeinem der Ansprüche 1 bis 8 definiert.

12. Vakzin, welches die chimäre Sequenz wie in irgendeinem der Ansprüche 1 bis 8 definiert umfasst oder daraus besteht oder den Vektor wie in Anspruch 9 definiert umfasst oder daraus besteht oder das chimäre Protein wie in Anspruch 11 definiert umfasst oder daraus besteht.

13. Chimäre Sequenz wie in irgendeinem der Ansprüche 1 bis 8 definiert oder Vektor wie in Anspruch 9 definiert oder chimäres Protein wie in Anspruch 11 definiert zur Verwendung bei der Behandlung und Prävention von HPV-verursachten Infektionen und/oder Komplikationen.

14. Chimäre Sequenz wie in irgendeinem der Ansprüche 1 bis 8 definiert oder Vektor wie in Anspruch 9 definiert oder chimäres Protein wie in Anspruch 11 definiert zur Verwendung nach Anspruch 13, wobei die HPV-verursachten Infektionen und/oder Komplikationen aus der Gruppe ausgewählt sind, welche aus Mandelkarzinom, Peniskarzinom, Kopf-Hals-Karzinom, Gebärmutterkarzinom, intraepithelialer zervikaler Neoplasie besteht.

15. Ribosomen-inhibierendes Protein (RIP), das durch Mutagenese nicht-toxisch gemacht wurde, in Assoziation mit einem Antigen zur Verwendung bei der Adjuvanz-Behandlung zur Erhöhung der Immunogenität des Antigens, wobei das Ribosomen-inhibierende Protein (RIP) Saporin ist.

## Revendications

1. Séquence chimère comprenant ou composée d'au moins une séquence nucléotidique codant pour un antigène de papillomavirus humain (HPV) tumoral rendu non oncogène par mutagenèse et d'une séquence nucléotidique codant pour une protéine inhibitrice des ribosomes (RIP) rendue non toxique par mutagenèse, dans laquelle la séquence, au moins au nombre de une, codant pour un antigène de papillomavirus humain tumoral est condensée au niveau de l'extrémité 3' et/ou 5' de ladite séquence codant pour une protéine inhibitrice des ribosomes, ladite protéine inhibitrice des ribosomes (RIP) étant la saporine.

2. Séquence selon la revendication 1, dans laquelle la séquence codant pour un antigène de papillomavirus humain tumoral est sélectionnée parmi celles codant pour E7, E6, E2, E5, L2, L1, de préférence E7 et E6, HPV16 et HPV18, ou des fragments de celles-ci.

3. Séquence selon l'une quelconque des revendications précédentes, dans laquelle la séquence codant pour un antigène de papillomavirus humain (HPV) tumoral est mutée au niveau du site de liaison au rétinoblastome.

4. Séquence selon la revendication 3, dans laquelle la séquence codant pour un antigène de papillomavirus humain tumoral muté est le gène E7, dans lequel le motif DLYCYE (ID SEQ. n° 4) a été muté en séquence GLYGYG (ID SEQ. n° 6).

5. Séquence selon l'une quelconque des revendications précédentes, dans laquelle la séquence codant pour la saporine est mutée dans des acides aminés invariants de site catalytique de façon telle qu'il en résulte une perte d'activité catalytique.

6. Séquence selon la revendication 5, dans laquelle les acides aminés acide glutamique 176 et arginine 179 de la saporine sont mutés en lysine et glutamine respectivement.

7. Séquence selon l'une quelconque des revendications précédentes, comprenant en outre au moins une séquence nucléotidique codant pour un peptide signal situé en extrémité 5' de ladite séquence chimère.

8. Séquence selon la revendication 7, dans laquelle le peptide signal est sélectionné dans le groupe composé d'un peptide signal dirigeant vers le réticulum endoplasmique de SAP apoplastique (SAPss) ou pour la sécrétion d'autres protéines végétales.

9. Vecteur comprenant la séquence selon l'une quelconque des revendications précédentes.

10. Cellule hôte comprenant le vecteur selon la revendication 9.

11. Protéine chimère codée par la séquence chimère selon l'une quelconque des revendications 1 à 8.

12. Vaccin comprenant ou composé de la séquence chimère selon l'une quelconque des revendications 1 à 8, ou comprenant ou composé du vecteur selon la revendication 9, ou comprenant ou composé de la protéine chimère selon la revendication 11.

13. Séquence chimère selon l'une quelconque des revendications 1 à 8, ou vecteur selon la revendication 9, ou protéine chimère selon la revendication 11, à utiliser dans le traitement et la prévention d'infections et/ou de complications dérivant du HPV.

14. Séquence chimère selon l'une quelconque des revendications 1 à 8, ou vecteur selon la revendication 9, ou protéine chimère selon la revendication 11, à utiliser selon la revendication 13, où les infections et/ou complications dérivant du HPV sont sélectionnées dans le groupe composé du carcinome de l'amygdale, du cancer du pénis, du cancer de la tête et du cou, du cancer de l'utérus, de la néoplasie cervicale intraépithéliale.

15. Protéine inhibitrice des ribosomes (RIP) rendue non toxique par mutagenèse en association avec un antigène, à utiliser dans le traitement adjuvant destiné à augmenter l'immunogénicité dudit antigène, où ladite protéine inhibitrice des ribosomes (RIP) est la saporine.
